# EUROPÄISCHE PATENTSCHRIFT

(11) **EP 2 673 016 B1**
(45) Veröffentlichungstag und Bekanntmachung des Hinweises auf die Patenterteilung: **03.02.2016**
(21) Anmeldenummer: 12718985.0
(22) Anmeldetag: 03.05.2012
(51) Int. Cl.: A61M 1/00

(54) **VORRICHTUNG ZUR BEREITSTELLUNG VON UNTERDRUCK ZUR UNTERDRUCKBEHANDLUNG VON WUNDEN MIT EINER HALTE- ODER TRAGEINRICHTUNG**
DEVICE FOR PROVIDING A VACUUM FOR THE VACUUM TREATMENT OF WOUNDS, COMPRISING A HOLDING OR CARRYING DEVICE
DISPOSITIF DESTINÉ À FOURNIR UNE PRESSION NÉGATIVE PERMETTANT DE TRAITER DES PLAIES SOUS PRESSION NÉGATIVE, POURVU D'UN SYSTÈME DE MAINTIEN OU DE SUPPORT

(30) Priorität: 01.06.2011 DE 102011076868
(43) Veröffentlichungstag der Anmeldung: 18.12.2013
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE)
(74) Vertreter: DREISS Patentanwälte PartG mbB
(86) Internationale Anmeldenummer: PCT/EP2012/058064
(87) Internationale Veröffentlichungsnummer: WO 2012/163617

(56) Entgegenhaltungen:
- WO-A2-2007/030599
- US-A- 6 038 745
- US-A1- 2002 008 125
- US-A1- 2010 187 065

## Beschreibung

Die Erfindung betrifft eine tragbare Vorrichtung zur Bereitstellung von Unterdruck zur medizinischen Unterdruckbehandlung von Wunden am menschlichen oder tierischen Körper, umfassend eine unterdruckerzeugende Saugpumpe in einem Gehäuseteil der Vorrichtung, wobei ein Anschluss für eine zum Körper führende Saugleitung vorgesehen ist, sodass eine Unterdruckkommunikation zwischen der Saugpumpe und der zum Körper führenden Saugleitung herstellbar ist, und eine Halte- oder Trageinrichtung mit zwei an dem Gehäuseteil angeordneten, gehäuseseitigen Verbindungsabschnitten zur Verbindung mit zwei gurtseitigen Verbindungsabschnitten eines Halte- oder Tragegurts (vergleiche US 2010/187065A1). Aus der US 2002/0008125 A1 ist ein Tragegurtsystem bekannt.

Wenn vorstehend von einer tragbaren Vorrichtung die Rede ist, so bedeutet dies, dass der Patient die Vorrichtung mitführen kann, sodass er mobil ist und dennoch seine Wunde dauerhaft, d.h. ohne Unterbrechung, therapiert werden kann. Die tragbare Vorrichtung kann dabei mittels einer Halte- oder Trageinrichtung am Körper des Patienten gehalten und mitgeführt werden. Eine tragbare Vorrichtung der hier in Rede stehenden Art kann aber natürlich auch im stationären Betrieb, also losgelöst vom Körper des Patienten, eingesetzt werden; sie kann solchenfalls beispielsweise an einem Pflegebett befestigt oder neben dem Pflegebett abgestellt werden.

Vorrichtungen zur Unterdruckwundbehandlung sind bereits mehrfach beschrieben worden, insbesondere durch US 2004/0073151 A1, WO 2009/047524 A2, WO 2007/030599 A2 oder EP 1 905 465 A1, EP 777 504 B und durch DE 10 2009 038 130 A1 und DE 10 2009 038 131 A1 der Anmelderin. In den beiden letztgenannten Veröffentlichungen ist bereits eine tragbare Vorrichtung beschrieben, wobei ein gurtförmiger Abschnitt als Halte- oder Trageinrichtung für den mobilen Betrieb erwähnt ist, wobei diese Halte- oder Trageinrichtung für den stationären Betrieb des Geräts abgenommen werden kann.

Bei derartigen Vorrichtungen zur Unterdruckbehandlung von Wunden kommuniziert eine Saugpumpe über eine Saugleitung mit der Wunde oder der Wundumgebung, wobei ein Wundverband mit einem luftundurchlässigen Abdeckmaterial zum luftdichten Verschließen der Wunde und der Wundumgebung vorgesehen ist, sodass ein Unterdruck im Wundraum herstellbar und Flüssigkeiten aus dem Wundraum absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Eine gewisse Nachgiebigkeit des Wundverbands und Abdeckmaterials ist jedoch typischerweise gegeben. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mmHg (mm Quecksilbersäule) (1 mm Hg = 1 Torr. = 133,322 Pa). Dieser Unterdruckbereich bis höchstens 250 mmHg hat sich als für die Wundheilung geeignet erwiesen. Ein bevorzugter Unterdruckbereich liegt zwischen 10 und 150 mmHg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit der Unterdruck erzeugenden Einrichtung kommuniziert, vorzugsweise unter Zwischenschaltung eines Behälters zur Aufnahme von Körperflüssigkeiten, insbesondere von aus einer Wunde abgesaugten Wundsekreten.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Der vorliegenden Erfindung liegt, ausgehend von einer gattungsgemäßen tragbaren Vorrichtung zur Bereitstellung von Unterdruck zur medizinischen Unterdruckbehandlung von Wunden die Aufgabe zugrunde, die Handhabbarkeit der Vorrichtung im stationären und im mobilen Betrieb weiter zu verbessern.

Diese Aufgabe wird bei einer gattungsgemäßen Vorrichtung erfindungsgemäß dadurch gelöst, dass der Halte- oder Tragegurt beabstandet zu den sich gegenüberliegenden Enden des Halte- oder Tragegurts und den dort jeweils angeordneten ersten und zweiten gurtseitigen Verbindungsabschnitten und zwischen diesen Enden angeordnet einen dritten gurtseitigen Verbindungsabschnitt zur Verbindung mit einem der gehäuseseitigen Verbindungsabschnitte und/oder mit einem der endseitig angeordneten gurtseitigen Verbindungsabschnitte aufweist und dadurch, dass die Vorrichtung eine Verbindungseinrichtung zur Verbindung des Halte- oder Tragegurts mit einem externen Träger, insbesondere einem Haltegestell, aufweist.

Die erfindungsgemäße Vorrichtung ermöglicht es, den Halte- oder Tragegurt in konventioneller Weise zu verwenden, nämlich durch Nutzung von zwei gurtseitigen Verbindungsabschnitten, welche an den Enden des Halte- oder Tragegurts angeordnet sind und mit den gehäuseseitigen Verbindungsabschnitten verbindbar sind. Hierdurch kann eine maximale Länge des Halte- oder Tragegurts genutzt werden, vorzugsweise dann, wenn ein Patient die tragbare Vorrichtung mit sich führt.

Der dritte gurtseitige Verbindungsabschnitt ermöglicht es, die nutzbare Länge des Halte- oder Tragegurts in einfacher Weise zu verkürzen, nämlich indem einer der endseitig angeordneten gurtseitigen Verbindungsabschnitte zur Verbindung mit einem der gehäuseseitigen Verbindungsabschnitte genutzt wird und indem der dritte gurtseitige Verbindungsabschnitt zur Verbindung mit dem anderen gehäuseseitigen Verbindungsabschnitt und/oder zur Verbindung mit dem anderen endseitig angeordneten gurtseitigen Verbindungsabschnitt genutzt wird.

Die wirksame Länge des Halte- oder Tragegurts kann beispielsweise dadurch verändert werden, dass ein endseitig angeordneter erster oder zweiter gurtseitiger Verbindungsabschnitt von einem gehäuseseitigen Abschnitt gelöst und an dessen Stelle der dritte gurtseitige Verbindungsabschnitt mit dem freigewordenen gehäuseseitigen Verbindungsabschnitt verbunden wird.

Die wirksame Länge des Halte- oder Tragegurts kann - ausgehend von einem Zustand, in welchem beide endseitig angeordnete, gurtseitige Verbindungsabschnitte mit beiden gehäuseseitigen Verbindungsabschnitten verbunden sind - auch dadurch verändert werden, dass zusätzlich auch der dritte gurtseitige Verbindungsabschnitt mit einem der beiden gehäuseseitigen Verbindungsabschnitte verbunden wird, dass der dritte gurtseitige Verbindungsabschnitt, mit einem der beiden endseitig angeordneten, gurtseitigen Verbindungsabschnitte verbunden wird oder dass der dritte gurtseitige Verbindungsabschnitt sowohl mit einem der beiden gehäuseseitigen Verbindungsabschnitte als auch mit einem der beiden endseitig angeordneten, gurtseitigen Verbindungsabschnitte verbunden wird.

Die Verkürzung der wirksamen Länge des Halte- oder Tragegurts ist zum Beispiel vorteilhaft, um die tragbare Vorrichtung von Hand zu tragen. In diesem Zustand ist das Gehäuse der tragbaren Vorrichtung dank der Verkürzung des Halte- oder Tragegurts näher an der tragenden Hand eines Patienten oder des medizinischen Personals angeordnet. Die verkürzte Halte- oder Tragegurtlänge kann insbesondere aber auch genutzt werden, um die tragbare Vorrichtung an einem externen Träger, beispielsweise an einem Gestell, insbesondere einem Pflegebett, zu befestigen, ohne dabei darauf achten zu müssen, dass das Gehäuse der tragbaren Vorrichtung zu weit in Bodennähe kommt, was mit ungewünschten Bein- oder Fußkontakten oder auch einer Verschmutzung einhergehen könnte.

Bei einer bevorzugten Ausführungsform der Erfindung ist vorgesehen, dass ein erster Abstand zwischen dem ersten gurtseitigen Verbindungsabschnitt und dem dritten gurtseitigen Verbindungsabschnitt in einem ersten Gurtabschnitt kürzer ist als ein zweiter Abstand zwischen dem dritten gurtseitigen Verbindungsabschnitt und dem zweiten gurtseitigen Verbindungsabschnitt in einem zweiten Gurtabschnitt. Dies hat den Vorteil, dass die maximale Länge des Halte- oder Tragegurts bei Nutzung des ersten gurtseitigen Verbindungsabschnitts und des zweiten gurtseitigen Verbindungsabschnitts wahlweise auf einen ersten (kleineren) Abstand oder einen zweiten (größeren) Abstand verkürzt werden kann.

Der Abstand zwischen dem ersten und dem zweiten gurtseitigen Verbindungsabschnitt beträgt vorzugsweise zwischen ungefähr 50 und 160 cm.

Der Abstand zwischen dem ersten und dem dritten gurtseitigen Verbindungsabschnitt beträgt vorzugsweise zwischen ungefähr 10 cm und 40 cm.

Der Abstand zwischen dem zweiten gurtseitigen Verbindungsabschnitt und dem dritten gurtseitigen Verbindungsabschnitt beträgt vorzugsweise zwischen ungefähr 40 cm und 120 cm.

Besonders bevorzugt ist es, wenn die Länge des ersten Gurtabschnitts unveränderbar ist. Hierdurch wird die Handhabung des Halte- oder Tragegurts weiter vereinfacht.

Ferner ist es bevorzugt, wenn eine Einstelleinrichtung zur Einstellung der Länge des zweiten Gurtabschnitts vorgesehen ist. Hierdurch ist es möglich, die wirksame Länge des Halte- oder Tragegurts anzupassen, insbesondere wenn die tragbare Vorrichtung in konventioneller Weise am Körper eines Patienten mitgeführt werden soll.

Erfindungsgemäß ist eine Verbindungseinrichtung zur Verbindung des Halte- oder Tragegurts mit einem externen Träger, insbesondere einem Haltegestell, vorgesehen. Die Verbindungseinrichtung ermöglicht eine besonders zuverlässige Fixierung der tragbaren Vorrichtung an dem externen Träger, und zwar derart, dass die Verbindungseinrichtung sowohl mit dem externen Träger als auch mit dem Halte- oder Tragegurt verbunden ist. Der Halte- oder Tragegurt ist seinerseits mittels mindestens zwei gurtseitigen Verbindungsabschnitten mit den gehäuseseitigen Verbindungsabschnitten des Gehäuseteils verbunden.

Eine besonders einfache Handhabung der Verbindungseinrichtung ergibt sich, wenn diese eine Steck- und/oder Rastverbindung umfasst.

Bei einer bevorzugten Ausführungsform umfasst die Verbindungseinrichtung eine umfangseitig lösbar verschließbare Schlaufe. Dies ermöglicht eine einfach herstellbare umschließende Anordnung der Schlaufe an einem externen Träger, beispielsweise an einem Bettgestell.

Vorzugsweise ist die Verbindungseinrichtung zwischen dem ersten gurtseitigen Verbindungsabschnitt und dem dritten gurtseitigen Verbindungsabschnitt angeordnet, insbesondere, wenn der Abstand zwischen diesen Verbindungsabschnitten kleiner ist als der Abstand zwischen dem zweiten gurtseitigen Verbindungsabschnitt und dem dritten gurtseitigen Verbindungsabschnitt.

Besonders bevorzugt ist es, wenn die Verbindungseinrichtung unabhängig von einem Verbindungszustand der gurtseitigen Verbindungsabschnitte und der gehäuseseitigen Verbindungsabschnitte betätigbar ist. Somit ist es möglich, zunächst den Halte- oder Tragegurt mit dem Gehäuseteil der Vorrichtung zu verbinden und anschließend mittels der Verbindungseinrichtung eine Verbindung zu einem externen Träger zu schaffen. Es ist aber auch möglich, die Verbindungseinrichtung zunächst mit dem externen Träger zu verbinden, um dann anschließend zwei von den drei gurtseitigen Verbindungsabschnitten mit den gehäuseseitigen Abschnitten des Gehäuseteils zu verbinden.

Bei einer Ausführungsform der Erfindung ist vorgesehen, dass der Halte- oder Tragegurt einen Halte- oder Tragegriff umfasst, welcher vorzugsweise zwischen dem ersten gurtseitigen Verbindungsabschnitt und dem dritten gurtseitigen Verbindungsabschnitt angeordnet ist. Hierdurch wird der Tragekomfort der Vorrichtung weiter verbessert. Der Halte- oder Tragegriff ist beispielsweise in Form einer gepolsterten Lasche ausgebildet.

Ferner ist es bevorzugt, wenn der Halte- oder Tragegurt eine Schulterauflage umfasst, welche vorzugsweise zwischen dem zweiten gurtseitigen Verbindungsabschnitt und dem dritten gurtseitigen Verbindungsabschnitt angeordnet ist. Auch hierdurch wird der Tragekomfort der Vorrichtung weiter verbessert.

Eine besonders einfache Handhabung der tragbaren Vorrichtung ergibt sich, wenn die gurtseitigen Verbindungsabschnitte in Form von selbsttätig schließenden Haken (beispielsweise mittels Federn vorgespannte Karabiner) ausgebildet sind.

Eine besonders zuverlässige Fixierung des Halte- oder Tragegurts an dem Gehäuseteil ergibt sich, wenn die gehäuseseitigen Verbindungsabschnitte in Form von Ösen ausgebildet sind.

Bevorzugte Ausführungsformen der erfindungsgemäßen Vorrichtung weisen ferner eines oder mehrere der folgenden Merkmale auf:
- es ist ein Behälter zur Aufnahme von Körperflüssigkeiten, insbesondere von aus einer Wunde abgesaugten Wundsekreten, vorgesehen;
- der Behälter ist lösbar an dem Gehäuseteil der Vorrichtung befestigbar;
- der Behälter ist im befestigten Zustand von der Saugpumpe mit Unterdruck beaufschlagbar;
- die Unterdruckkommunikation zwischen der Saugpumpe und der zum Körper führenden Saugleitung ist unter Zwischenschaltung des Behälters herstellbar;
- es ist ein Drucksensor vorgesehen, welcher zur Messung des Drucks vorzugsweise in einem Leitungsabschnitt zwischen dem Behälter und der Saugpumpe angeordnet ist;
- es ist eine programmierbare elektronische Steuereinrichtung vorgesehen, welche mindestens unter Berücksichtigung von vorgegebenen und/oder vorgebbaren Parametern und von durch den Drucksensor gemessenen Druckwerten die Saugpumpe ansteuern kann;
- es ist eine Spüleinrichtung zum Spülen einer mit Unterdruck zu beaufschlagenden Wunde vorgesehen.

Weitere Merkmale und Vorteile der Erfindung sind Gegenstand der nachfolgenden Beschreibung und der zeichnerischen Darstellung bevorzugter Ausführungsbeispiele.

In den Zeichnungen zeigen:

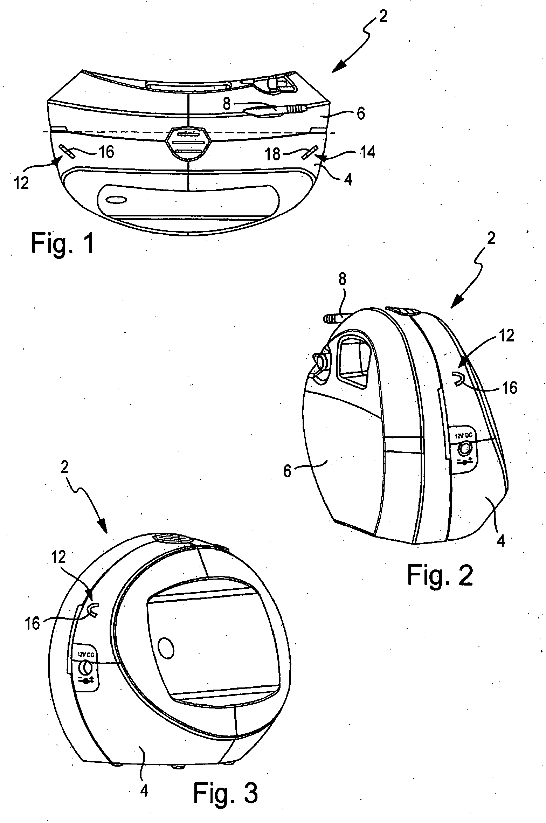

Fig. 1 eine Draufsicht auf eine Ausführungsform eines Gehäuseteils einer tragbaren Vorrichtung;
Fig. 2 eine perspektivische Ansicht des Gehäuseteils gemäß Fig. 1 aus einer rückwärtigen Perspektive;
Fig. 3 eine perspektivische Ansicht des Gehäuseteils gemäß Fig. 1 aus einer vorderen Perspektive;
Fig. 4 eine perspektivische Ansicht einer Ausführungsform eines mit dem Gehäuseteil lösbar verbindbaren Halte- oder Tragegurts;
Fig. 5 einen in Fig. 4 mit V bezeichneten Ausschnitt in vergrößerter Darstellung;
Fig. 6 einen in Fig. 4 mit VI bezeichneten Ausschnitt in vergrößerter Darstellung;
Fig. 7 einen in Fig. 4 mit VII bezeichneten Ausschnitt in vergrößerter Darstellung; und
Fig. 8 einen in Fig. 4 mit VIII bezeichneten Ausschnitt in vergrößerter Darstellung.

Eine Ausführungsform einer tragbaren Vorrichtung ist in der Zeichnung insgesamt mit dem Bezugszeichen 2 bezeichnet. Die Vorrichtung 2 umfasst ein Gehäuseteil 4, in welchem eine (nicht dargestellte) Saugpumpe angeordnet ist.

Das Gehäuseteil 4 ist mit einem Behälter 6 zur Aufnahme von Körperflüssigkeiten lösbar verbunden. Der Behälter 6 ist mittels eines Anschlusses 8 mit einer zum Körper führenden Saugleitung verbindbar, so dass in einem Wundbereich eines Körpers anliegende Körperflüssigkeit mittels der Saugpumpe abgesaugt und in den Behälter 6 eingeleitet werden kann.

Die Vorrichtung 2 umfasst weitere Bauteile, beispielsweise einen Drucksensor zur Messung des Drucks in einem Leitungsabschnitt zwischen dem Behälter und der Saugpumpe und eine programmierbare elektronische Steuereinrichtung, welche mindestens unter Berücksichtung von vorgegebenen und/oder vorgebbaren Parametern und von durch den Drucksensor gemessenen Druckwerten die Saugpumpe ansteuern kann. Hinsichtlich der Anordnung, des Aufbaus und der Funktionsweise dieser Bauteile sowie weiterer Bauteile der Vorrichtung 2 wird auf die Offenbarung der DE 10 2009 038 130 A1 in vollem Umfang Bezug genommen.

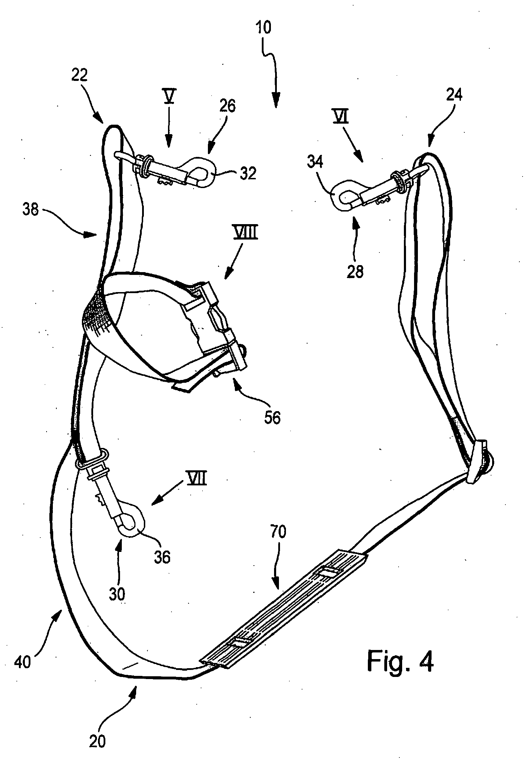

Die Vorrichtung 2 umfasst ferner eine Halte- oder Trageinrichtung, welche in Figur 4 dargestellt ist und dort insgesamt mit dem Bezugszeichen 10 bezeichnet ist.

Das Gehäuseteil 4 weist zur Verbindung mit der Halte- oder Trageinrichtung 10 zwei gehäuseseitige Verbindungsabschnitte 12, 14 auf. Die gehäuseseitigen Verbindungsabschnitte sind in Form von Ösen 16, 18 ausgebildet.

Die gehäuseseitigen Verbindungsabschnitte 12, 14 sind relativ zueinander beabstandet und vorzugsweise in einem in Gebrauchslage oberen Bereich des Gehäuseteils 4 angeordnet.

Die Halte- oder Trageinrichtung 10 umfasst einen Halte- oder Tragegurt 20, welcher zwei einander gegenüberliegende Enden 22, 24 aufweist. An dem ersten Ende 22 des Halte- oder Tragegurts 20 ist ein erster gurtseitiger Verbindungsabschnitt 26 angeordnet. An dem zweiten Ende des Halte- oder Tragegurts 20 ist ein zweiter gurtseitiger Verbindungsabschnitt 28 angeordnet.

Entlang des Verlaufs des Halte- oder Tragegurts 20 gesehen ist zwischen den Enden 22 und 24 ein weiterer Verbindungsabschnitt in Form eines dritten gurtseitigen Verbindungsabschnitts 30 vorgesehen.

Die gurtseitigen Verbindungsabschnitte 26, 28 und 30 sind insbesondere in Form selbsttätig schließender Haken 32, 34, 36 ausgebildet (vergleiche Figuren 4 bis 7).

Der Halte- oder Tragegurt 20 weist einen ersten Gurtabschnitt 38 auf, welcher sich zwischen dem ersten gurtseitigen Verbindungsabschnitt 26 und dem dritten gurtseitigen Verbindungsabschnitt 30 erstreckt. Der Halte- oder Tragegurt 20 weist ferner einen zweiten Gurtabschnitt 40 auf, welcher sich zwischen dem dritten gurtseitigen Verbindungsabschnitt 30 und dem zweiten gurtseitigen Verbindungsabschnitt 28 erstreckt.

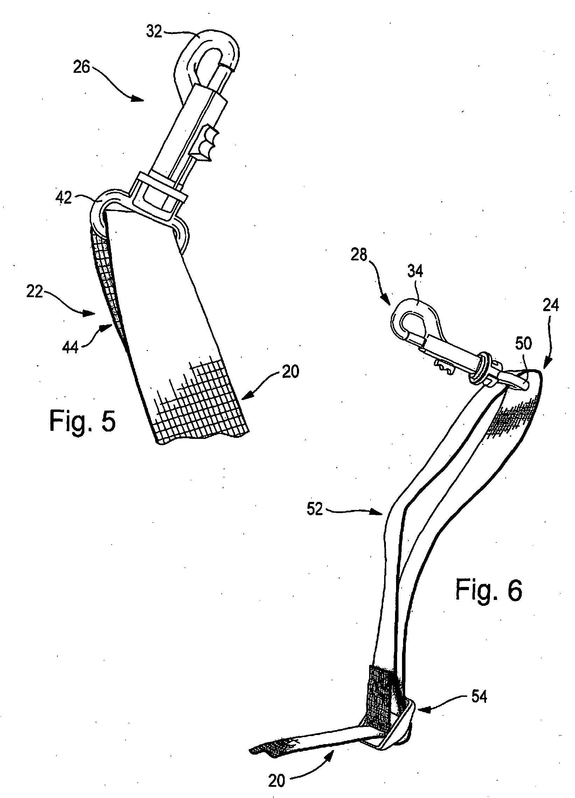

Die Länge des ersten Gurtabschnitts 38 definiert einen maximalen Abstand zwischen dem ersten gurtseitigen Verbindungsabschnitt 26 und dem dritten gurtseitigen Verbindungsabschnitt 30. Diese Länge ist bei einem bevorzugten Ausführungsbeispiel nicht veränderbar. Hierfür ist der erste gurtseitige Verbindungsabschnitt 26 mittels eines ringförmigen Halteabschnitts 42 an einer endseitigen Umschlaufung 44 des Halte- oder Tragegurts 20 befestigt (vergleiche Figur 5).

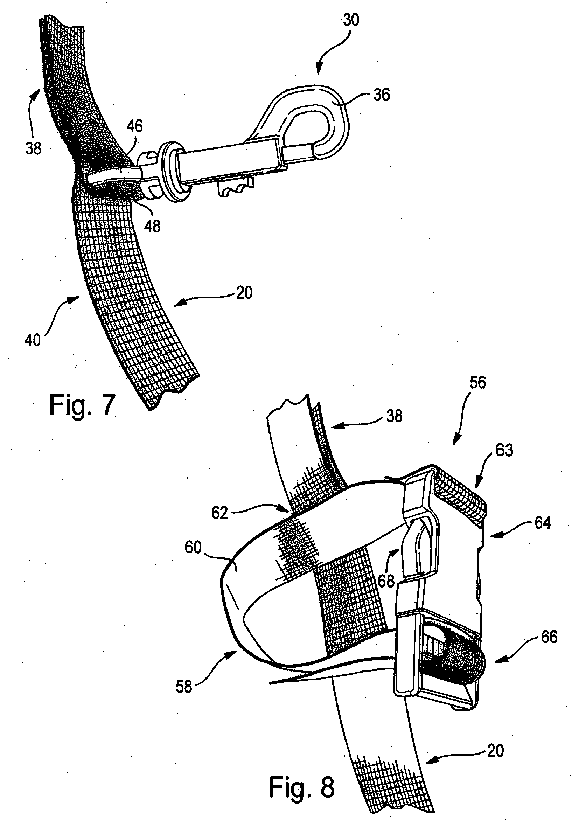

Der dritte gurtseitige Verbindungsabschnitt 30 weist ebenfalls einen ringförmigen Halteabschnitt 46 auf, welcher an einer Umschlaufung 48 des Halte- oder Tragegurts 20 gesichert ist (vergleiche Figur 7).

Die Geometrie und insbesondere die Schlaufenlänge der Umschlaufungen 44 und 48 ist nicht veränderbar. Hingegen ist der zweite gurtseitige Verbindungsabschnitt 28 (vergleiche Figur 6) mittels eines ringförmigen Halteabschnitts 50 an einer Umschlaufung 52 gesichert, deren Länge mittels einer Einstelleinrichtung 54 einstellbar ist. Durch Wahl der Länge der Umschlaufung 52 ist auch die Länge des zweiten Gurtabschnitts 40 und somit die Gesamtlänge des Halte- oder Tragegurts 20 einstellbar.

In dem ersten Gurtabschnitt 38 ist eine insgesamt mit dem Bezugszeichen 56 bezeichnete Verbindungseinrichtung zur Verbindung des Halte- oder Tragegurts 20 mit einem ((nicht dargestellten) externen Träger angeordnet (vergleiche Figuren 4 und 8). Die Verbindungseinrichtung 56 umfasst eine umfangsseitig lösbar verschließbare Schlaufe 58.

Die Schlaufe 58 umfasst ein Band 60, welches vorzugsweise quer, insbesondere senkrecht, zu dem Halte- oder Tragegurt 20 angeordnet ist und insbesondere in einem Verbindungsbereich 62 unlösbar mit dem Halte- oder Tragegurt 20 verbunden ist. Das Band 60 weist ein erstes Bandende 63 zur Verbindung mit einer Rastaufnahme 64 und ein zweites Bandende 66 zur Verbindung mit einem Raststecker 68 auf. Der Raststecker 68 ist rastend und lösbar in der Rastaufnahme 64 aufnehmbar.

Die wirksame Länge der Schlaufe 58 ist einstellbar, indem die Position des zweiten Bandendes 66 relativ zu dem Raststecker 68 wählbar ist (optional oder zusätzlich hierzu ist die Position des ersten Bandendes 64 relativ zu der Rastaufnahme 64 wählbar).

Während des Gebrauchs der Vorrichtung 2 kann diese mittels der Halte-oder Trageinrichtung 10 am Körper eines Patienten mitgeführt werden. Beispielsweise können die ersten und zweiten Verbindungsabschnitte 26 und 28 mit den gehäuseseitigen Verbindungsabschnitten 12 und 14 verbunden werden. In diesem Fall ist die wirksame Länge des Halte- oder Tragegurts 20 gleich dem Abstand zwischen den endseitig angeordneten, gurtseitigen Verbindungsabschnitten 26 und 28. Dabei kann zur weiteren Verbesserung des Tragekomforts eine beispielsweise gummierte Schulterauflage 70, welche verschiebbar entlang des Halte- oder Tragegurts 20 angeordnet ist, so positioniert werden, dass sie auf einer Schulter eines Patienten aufliegt.

Bei einer weiteren Nutzungsart der tragbaren Vorrichtung 2 werden die gurtseitigen Verbindungsabschnitte 26 und 30 mit den gehäuseseitigen Verbindungsabschnitten 12 und 14 verbunden, oder es wird der dritte gurtseitige Verbindungsabschnitt 30 mit dem zweiten gurtseitigen Verbindungsabschnitt 28 verbunden, während dieser mit einem der gehäuseseitigen Verbindungsabschnitte 12 oder 14 verbunden ist. Dies ermöglicht es, den ersten Gurtabschnitt 38 als Tragegriff zum Tragen des Gehäuseteils 4 zu verwenden. Optional ist in dem ersten Gurtabschnitt 38 ein zusätzlicher, insbesondere gepolsteter Hand- oder Tragegriff (nicht dargestellt) angeordnet.

Schließlich es möglich, das Gehäuseteil 4 mittels der Verbindungseinrichtung 56 mit einem externen Träger zu verbinden. Hierfür wird die Schlaufe 58 um ein Gestellteil eines externen Trägers herum angeordnet. Bevorzugt ist es hierbei, wenn die wirksame Länge des Halte- oder Tragegurts 20 gleich der Länge des ersten Gurtabschnitts 38 ist (vergleiche vorstehenden Absatz). Hierdurch ergibt sich ein relativ kurzer Abstand zwischen dem externen Träger und dem Gehäuseteil 4.

## Patentansprüche

1. Tragbare Vorrichtung (2) zur Bereitstellung von Unterdruck zur medizinischen Unterdruckbehandlung von Wunden am menschlichen oder tierischen Körper, umfassend eine unterdruckerzeugende Saugpumpe in einem Gehäuseteil (4) der Vorrichtung, wobei ein Anschluss (8) für eine zum Körper führende Saugleitung vorgesehen ist, sodass eine Unterdruckkommunikation zwischen der Saugpumpe und der zum Körper führenden Saugleitung herstellbar ist, und eine Halte- oder Trageinrichtung (10) mit zwei an dem Gehäuseteil (4) angeordneten, gehäuseseitigen Verbindungsabschnitten (12, 14) zur Verbindung mit zwei gurtseitigen Verbindungsabschnitten (26, 28) eines Halte- oder Tragegurts (20), **dadurch gekennzeichnet, dass** der Halte- oder Tragegurt (20) beabstandet zu den sich gegenüberliegenden Enden (22, 24) des Halte- oder Tragegurts (20) und den dort jeweils angeordneten ersten und zweiten gurtseitigen Verbindungsabschnitten (26, 28) und zwischen diesen Enden (22, 24) angeordnet einen dritten gurtseitigen Verbindungsabschnitt (30) zur Verbindung mit einem der gehäuseseitigen Verbindungsabschnitte (12, 14) und/oder mit einem der endseitig angeordneten gurtseitigen Verbindungsabschnitte (26, 28) aufweist und **gekennzeichnet durch** eine Verbindungseinrichtung (56) zur Verbindung des Halte- oder Tragegurts (20) mit einem externen Träger, insbesondere einem Haltegestell.

2. Tragbare Vorrichtung (2) nach Anspruch 1, **dadurch gekennzeichnet, dass** ein erster Abstand zwischen dem ersten gurtseitigen Verbindungsabschnitt (26) und dem dritten gurtseitigen Verbindungsabschnitt (30) in einem ersten Gurtabschnitt (38) kürzer ist als ein zweiter Abstand zwischen dem dritten gurtseitigen Verbindungsabschnitt (30) und dem zweiten gurtseitigen Verbindungsabschnitt (28) in einem zweiten Gurtabschnitt (40).

3. Tragbare Vorrichtung (2) nach Anspruch 2, **dadurch gekennzeichnet, dass** die Länge des ersten Gurtabschnitts (38) unveränderbar ist.

4. Tragbare Vorrichtung (2) nach Anspruch 2 oder 3, **gekennzeichnet durch** eine Einstelleinrichtung (54) zur Einstellung der Länge des zweiten Gurtabschnitts (40).

5. Tragbare Vorrichtung (2) nach einem der voranstehenden Ansprüche, **dadurch gekennzeichnet, dass** die Verbindungseinrichtung (56) eine umfangsseitig lösbar verschließbare Schlaufe (58) umfasst.

6. Tragbare Vorrichtung (2) nach einem der voranstehenden Ansprüche, **dadurch gekennzeichnet, dass** die Verbindungseinrichtung (56) eine Steck- und/oder Rastverbindung umfasst.

7. Tragbare Vorrichtung (2) nach einem der voranstehenden Ansprüche, **dadurch gekennzeichnet, dass** die Verbindungseinrichtung (56) mit dem Halte- oder Tragegurt (20) verbunden, insbesondere unlösbar verbunden, ist.

8. Tragbare Vorrichtung (2) nach einem der voranstehenden Ansprüche, **dadurch gekennzeichnet, dass** die Verbindungseinrichtung (56) zwischen dem ersten gurtseitigen Verbindungsabschnitt (26) und dem dritten gurtseitigen Verbindungsabschnitt (30) angeordnet ist.

9. Tragbare Vorrichtung (2) nach einem der voranstehenden Ansprüche, **dadurch gekennzeichnet, dass** die Verbindungseinrichtung (56) unabhängig von einem Verbindungszustand der gurtseitigen Verbindungsabschnitte (26, 28, 30) und der gehäuseseitigen Verbindungsabschnitte (12, 14) betätigbar ist.

10. Tragbare Vorrichtung (2) nach einem der voranstehenden Ansprüche, **dadurch gekennzeichnet, dass** der Halte- oder Tragegurt (20) einen Halte- oder Tragegriff umfasst, welcher vorzugsweise zwischen dem ersten gurtseitigen Verbindungsabschnitt (26) und dem dritten gurtseitigen Verbindungsabschnitt (30) angeordnet ist.

11. Tragbare Vorrichtung (2) nach einem der voranstehenden Ansprüche, **dadurch gekennzeichnet, dass** der Halte- oder Tragegurt (20) eine Schulterauflage (70) umfasst, welche vorzugsweise zwischen dem zweiten gurtseitigen Verbindungsabschnitt (28) und dem dritten gurtseitigen Verbindungsabschnitt (30) angeordnet ist.

12. Tragbare Vorrichtung (2) nach einem der voranstehenden Ansprüche, **dadurch gekennzeichnet, dass** die gurtseitigen Verbindungsabschnitte (26, 28, 30) in Form von selbsttätig schließenden Haken (32, 34 36) ausgebildet sind.

13. Tragbare Vorrichtung (2) nach einem der voranstehenden Ansprüche, **dadurch gekennzeichnet, dass** die gehäuseseitigen Verbindungsabschnitte (12, 14) in Form von Ösen (16, 18) ausgebildet sind.

## Claims

1. Portable device (2) for providing a vacuum for medical vacuum treatment of wounds on the body of a person or of an animal, comprising a suction pump, which generates a vacuum and is located in a housing part (4) of the device, wherein a connection (8) is provided for a suction line that leads to the body, such that vacuum communication can be established between the suction pump and the suction line that leads to the body, and with a holding or carrying device (10) with two connecting sections (12, 14) on the housing, which are disposed on the housing part (4) for connection to two connecting sections (26, 28) on the strap of a holding or carrying strap (20), **characterized in that** the holding or carrying strap (20) comprises a third strap-side connecting section (30), which is spaced apart from the opposing ends (22, 24) of the holding or carrying strap (20) and from the first and second strap-side connecting sections (26, 28) disposed there, and arranged between these ends, for connection to one of the connecting sections (12, 14) on the housing and/or to a strap-side connecting section (26, 28) disposed at the end and **characterized by** a connecting device (56) for connecting the holding or carrying strap (20) to an external carrier, in particular, a holding frame.

2. Portable device (2) according to claim 1, **characterized in that** a first separation between the first connecting section (26) on the strap and the third connecting section (30) on the strap in a first strap section (38) is shorter than a second separation between the third connecting section (30) on the strap and the second connecting section (28) on the strap in a second strap section (40).

3. Portable device (2) according to claim 2, **characterized in that** the length of the first strap section (38) cannot be changed.

4. Portable device (2) according to claim 2 or 3, **characterized by** an adjustment device (54) for adjusting the length of the second strap section (40).

5. Portable device (2) according to one of the preceding claims, **characterized in that** the connecting device (56) has a loop (58) that can be closed and released on the peripheral side.

6. Portable device (2) according to one of the preceding claims, **characterized in that** the connecting device (56) comprises a plug and/or snap connection.

7. Portable device (2) according to one of the preceding claims, **characterized in that** the connecting device (56) is connected, in particular, in an undetachable fashion to the holding or carrying strap (20).

8. Portable device (2) according to one of the preceding claims, **characterized in that** the connecting device (56) is disposed between the first connecting section (26) on the strap and the third connecting section (30) on the strap.

9. Portable device (2) according to one of the preceding claims, **characterized in that** the connecting device (56) can be operated independently of a state of connection of the connecting sections (26, 28, 30) on the strap and the connecting sections (12, 14) on the housing.

10. Portable device (2) according to one of the preceding claims, **characterized in that** the holding or carrying strap (20) has a supporting strap or carrier handle which is advantageously disposed between the first connecting section (26) on the strap and the third connecting section (30) on the strap.

11. Portable device (2) according to one of the preceding claims, **characterized in that** the supporting strap or carrier handle (20) comprises a shoulder support (70) which is advantageously disposed between the second connecting section (28) on the strap and the third connecting section (30) on the strap.

12. Portable device (2) according to one of the preceding claims, **characterized in that** the connecting sections (26, 28, 30) on the strap are designed in the form of automatically closing hooks (32, 34, 36).

13. Portable device (2) according to one of the preceding claims, **characterized in that** the connecting sections (12, 14) on the housing are designed in the form of lugs (16, 18).

## Revendications

1. Dispositif portable (2) destiné à fournir de la pression négative pour le traitement médical par pression négative de plaies sur le corps humain ou animal, comprenant une pompe aspirante génératrice de pression négative située dans une partie de boîtier (4) du dispositif, un raccord (8) pour une conduite d'aspiration menant vers le corps étant prévu de sorte qu'une communication à pression négative peut être établie entre ladite pompe aspirante et ladite conduite d'aspiration menant vers le corps, ainsi qu'un dispositif de maintien ou de transport (10) ayant deux portions de liaison (12, 14) côté boîtier disposées sur la partie de boîtier (4) et destinées à être reliées à deux portions de liaison (26, 28) côté ceinture d'une ceinture de maintien ou de transport (20), **caractérisé par le fait que** ladite ceinture de maintien ou de transport (20) présente une troisième portion de liaison (30) côté ceinture qui est espacée des extrémités (22, 24) opposées de la ceinture de maintien ou de transport (20) et des première et deuxième portions de liaison (26, 28) côté ceinture qui y sont disposées respectivement, et qui est disposée entre ces extrémités (22, 24) et est destinée à être reliée à l'une des portions de liaison (12, 14) côté boîtier et/ou à l'une des portions de liaison (26, 28) côté ceinture disposées à l'extrémité, et **caractérisé par** un dispositif de liaison (56) pour relier la ceinture de maintien ou de transport (20) à un support externe, en particulier à un bâti de support.

2. Dispositif portable (2) selon la revendication 1, **caractérisé par le fait qu'**une première distance entre la première portion de liaison (26) côté ceinture et la troisième portion de liaison (30) côté ceinture dans une première section de ceinture (38) est inférieure à une deuxième distance entre la troisième portion de liaison (30) côté ceinture et la deuxième portion de liaison (28) côté ceinture dans une deuxième section de ceinture (40).

3. Dispositif portable (2) selon la revendication 2, **caractérisé par le fait que** la longueur de la première section de ceinture (38) est inchangeable.

4. Dispositif portable (2) selon la revendication 2 ou 3, **caractérisé par** un dispositif de réglage (54) pour régler la longueur de la deuxième section de ceinture (40).

5. Dispositif portable (2) selon l'une quelconque des revendications précédentes, **caractérisé par le fait que** le dispositif de liaison (56) comprend une boucle (58) apte à être fermée de manière amovible sur la circonférence.

6. Dispositif portable (2) selon l'une quelconque des revendications précédentes, **caractérisé par le fait que** ledit dispositif de liaison (56) comprend une liaison à emboîtement et/ou à encliquetage.

7. Dispositif portable (2) selon l'une quelconque des revendications précédentes, **caractérisé par le fait que** ledit dispositif de liaison (56) est relié à la ceinture de maintien ou de transport (20), en particulier de manière non détachable.

8. Dispositif portable (2) selon l'une quelconque des revendications précédentes, **caractérisé par le fait que** ledit dispositif de liaison (56) est disposé entre la première portion de liaison (26) côté ceinture et la troisième portion de liaison (30) côté ceinture.

9. Dispositif portable (2) selon l'une quelconque des revendications précédentes, **caractérisé par le fait que** ledit dispositif de liaison (56) peut être actionné indépendamment d'un état de liaison des portions de liaison (26, 28, 30) côté ceinture et des portions de liaison (12, 14) côté boîtier.

10. Dispositif portable (2) selon l'une quelconque des revendications précédentes, **caractérisé par le fait que** la ceinture de maintien ou de transport (20) comprend une poignée de maintien ou de transport qui, de préférence, est disposée entre la première portion de liaison (26) côté ceinture et la troisième portion de liaison (30) côté ceinture.

11. Dispositif portable (2) selon l'une quelconque des revendications précédentes, **caractérisé par le fait que** la ceinture de maintien ou de transport (20) comprend un appui-épaule (70) qui, de préférence, est disposé entre la deuxième portion de liaison (28) côté ceinture et la troisième portion de liaison (30) côté ceinture.

12. Dispositif portable (2) selon l'une quelconque des revendications précédentes, **caractérisé par le fait que** les portions de liaison (26, 28, 30) côté ceinture sont réalisées sous forme de crochets (32, 34, 36) se fermant automatiquement.

13. Dispositif portable (2) selon l'une quelconque des revendications précédentes, **caractérisé par le fait que** les portions de liaison (12, 14) côté boîtier sont réalisées sous forme d'oeillets (16, 18).
